Europäisches Patentamt

European Patent Office

Office européen des brevets

(11) Publication number: **0 205 844**
**B1**

(12) **EUROPEAN PATENT SPECIFICATION**

(45) Date of publication of patent specification: **04.07.90**

(21) Application number: **86106136.4**

(22) Date of filing: **05.05.86**

(51) Int. Cl.⁵: **C 12 Q 1/28**

(54) Diagnostic method for detecting periodontal disease.

(30) Priority: **06.05.85 US 730427**

(43) Date of publication of application:
**30.12.86 Bulletin 86/52**

(45) Publication of the grant of the patent:
**04.07.90 Bulletin 90/27**

(84) Designated Contracting States:
**AT BE CH DE FR GB IT LI LU NL SE**

(56) References cited:
**EP-A-0 036 563**
**EP-A-0 121 317**
**EP-A-0 158 796**
**WO-A-80/02596**
**US-A-3 691 018**
**US-A-4 334 540**

(73) Proprietor: **Richardson-Vicks, Inc.**
**Ten Westport Road**
**Wilton, CT 06897 (US)**

(72) Inventor: **Chaudhari, Panna R.**
**17 Spinning Wheel Road**
**Monroe Connecticut (US)**
Inventor: **Shah, Nutan B.**
**19 Reiner Drive**
**Shelton Connecticut (US)**

(74) Representative: **Vossius & Partner**
**Siebertstrasse 4 P.O. Box 86 07 67**
**D-8000 München 86 (DE)**

Courier Press, Leamington Spa, England.

**Description**

Background of the Invention
Periodontal diseases such as, for example, periodontitis, stomatitis, gingivitis and the like, are inflammatory conditions of the mouth characterized by inflammatory oral tissue changes usually due to local irritation. The destructive inflammatory process involves the interaction between bacteria, food debris, oral leukocytes and the epithelial attachment around the tooth and periodontal membrane, the result of which causes bone resorption (the supportive bone around the roots of the teeth) and inflammation of the tissues (gingiva) immediately surrounding human teeth involving the portion upward from the epithelial attachment (between the surface of gingiva and epithelial attachments only). The early detection of such inflammatory conditions is very important to proper dental treatment. Such detection is herein accomplished by taking advantage of the known fact that, at the site of inflammation, the body produces an increased number of leukocytes such as polymorphonuclear (PMN) leukocytes, which contain a peroxidase enzyme which is a good marker for detecting the presence of the inflammatory periodontal disease condition. When the inflammatory condition is present, the PMN leukocyte count will increase and, concommitantly, so will the peroxidase enzyme activity.

Detailed Description of Invention
The instant invention provides a simple saliva test for detecting the presence of inflammation due to periodontal disease. It takes advantage of the known chemical ability of peroxidase to catalyze the oxidation of a colorless hydrogen donor (i.e., a chromogen) to a colored product which can be determined colorimetrically. The subject peroxidatic test method is rapid and reliable with a strong positive correlation between the presence of peroxidase in the saliva sample and the existence of such disease.

The determination of peroxidase activity is herein achieved with a reagent mixture of hydrogen peroxide as the substrate and 4-aminoantipyrine as the chromogen in a buffered aqueous solution also containing a phenolic component as a coupling agent, said phenolic component being a member selected from the group consisting of (i) sodium 2-hydroxy-3,5-dichlorobenzene sulfonate; (ii) p-hydroxybenzoic acid; (iii) p-methoxyphenol; or (iv) 2,6-dichlorophenol. The 4-aminoantipyrine is oxidized by the hydrogen peroxide in the presence of peroxidase from the saliva sample and the intensity of the resultant color formation is proportional to the enzyme concentration and, accordingly, to the severity of the periodontal inflammation.

In our prior copending European Patent Application EP—A2 0 158 796 (Application No. 85 10 2113.9), a peroxidatic saliva test is described wherein phenol itself constitutes the coupling agent in the chromogen solution. Due to the physical properties of said phenol, however, such chromogen solution cannot be lyophilized as can the chromogen solutions of the present invention. The present invention, therefore, constitutes an improvement over said prior application.

For purposes of convenience, the following symbols will be used for each phenolic component, respectively:

|  |  |  |  |
|---|---|---|---|
| (i) | sodium 2-hydroxy-3,5-dichlorobenzene sulfonate (preferred): | HDCBS | |
| (ii) | p-hydroxybenzoic acid: | pHBA | |
| (iii) | p-methoxyphenol: | pMP | |
| (iv) | 2,6-dichlorophenol: | dCP | (outside the human) |

The reaction is carried out by mixing (outside the human) measured amounts of the chromogen and substrate solutions with a measured amount of saliva. The reaction is initiated upon contact of the hydrogen peroxide substrate with the saliva so that the order of mixing may first proceed by mixing the chromogen and substrate solutions to which mixture the saliva is then added, or, alternatively, by mixing the chromogen solution and saliva together and then adding the substrate solution.

The end point of the assay will obviously depend on the particular assay conditions employed. For example, increasing the concentration of chromogen and/or substrate, up to an optimum, will enhance the color formation, that is, the time required for observing the color produced by the reaction will be shortened. Thus, one can advantageously adjust the concentrations of chromogen and substrate within the given parameters such that the end point of the assay will be observable within a convenient period of time. Any such adjustment will be readily apparent and easily accomplished by the practitioner of the assay with some familiarization therewith.

Although minimal concentrations of chromogen and substrate may require longer assay times, for example, in excess of 1 hour, it has been generally found that use of the hereinafter indicated preferred concentrations to the maximum range concentrations will provide suitable assays in about 2 to about 60

EP 0 205 844 B1

minutes and, preferably, in about 3 to about 30 minutes, at room temperature (20—26°C), at which time the color intensity in the reaction mixture is observed. Slightly elevated temperatures up to about 45°C (i.e., below the inactivation temperature of the peroxidase enzyme) may also be employed to enhance the rate of reaction.

The lack of color formation indicates a normal condition, that is, an absence of PMN leukocytes in the saliva which, in turn, indicates the absence of periodontal disease. The formation of color, however, correlates to the presence of periodontal disease and the color intensity correlates to the severity of the disease. A convenient scoring system for each of the phenolic components, from 1 to 4, is as follows:

|  | HDCBS | pHBA | pMB | dCP |
|---|---|---|---|---|
| 1 = normal: | ——————no color developed—————— | | | |
| 2 = mild: | ———————light pink———————— | | | |
| 3 = moderate: | purplish pink | pinkish red | pinkish red | purplish pink |
| 4 = severe: | dark purple | dark red | dark red | dark purple |

The chromogen solution consists of the 4-amino-antipyrine chromogen, the phenolic coupling agent and appropriate buffers, preferably conventional alkali metal (sodium or potassium) phosphate buffers, to obtain a desired pH of about 4—9, preferably 6.8±0.05, for the reaction conditions.

### Chromogen Solution

|  | % w/v | |
|---|---|---|
| Components | Range | (pH - 6.8) Preferred |
| 4-Aminoantipyrine | 0.001-0.15 g | 0.017 g |
| Phenolic Component: | | |
| if HDCBS | 0.07-1.0 g | 0.42 g |
| if pHBA | 0.10-15.0 g | 1.5 g |
| if pMP | 4.1-130.0 mg | 25.0 mg |
| if dCP | 6.5-1000.0 mg | 65.0 mg |
| Potassium phosphate (monobasic) | 0.15-12.0 g | 1.5 g |
| Potassium phosphate (dibasic) | 0.15-13.0 g | 1.6 g |
| Water, q.s. | 100 ml | 100 ml |

The hydroperoxide substrate solution is a diluted aqueous solution of hydrogen peroxide.

### Substrate Solution

|  | % v/v | |
|---|---|---|
| Components | Range | Preferred |
| Hydrogen peroxide (30%) | 0.001-10 ml | 0.55 ml |
| Water, q.s. | 100 ml | 100.00 ml |

3

# EP 0 205 844 B1

In general, the method of this invention will utilize about 2—15 unit volumes of the chromogen solution and about 0.1—2 unit volumes of the substrate solution per unit volume of saliva. Optimum efficient amounts may vary, however, depending upon the concentrations of chromogen and/or substrate and the particular assay technique employed in the test. Such techniques, as more fully detailed hereinafter, include test tubes, filter pad sections, porcelain wells, spectrophotometers and the like. With the aforementioned preferred chromogen and substrate solutions we have found, for example, that about 2 unit volumes each of the substrate and chromogen solutions per unit volume of saliva is preferred when using test tubes or porcelain wells; that about 2 unit volumes of the chromogen solution and 1 unit volume of the substrate solution per unit volume of saliva is preferred when using filter pad sections (e.g. discs); and that about 15 unit volumes of the chromogen solution and 0.1 unit volume of the substrate solution per unit volume of saliva is preferred when using spectrophotometric instruments.

To obtain consistently reliable color formation, the pH and peroxide concentration must be controlled. As buffers, there can be used, for example, phosphate (preferred), phthalate, citrate, Tris, borate, succinate and the like buffers, the pH value and capacity thereof being selected in such a manner that the reaction mixture has a pH value of from about 4.0 to about 9.0, and preferably about 6.8. When mixing the chromogen solution and the peroxide substrate solution, it is advantageous to use from about $1.1 \times 10^{-4}$ mole to about 189 moles of 4-aminoantipyrine and from about $2.5 \times 10^{-3}$ mole to about $8.57 \times 10^2$ moles of HDCBS, or from about $5.93 \times 10^{-3}$ mole to about $2.14 \times 10^4$ moles of pHBA, or from about $3.13 \times 10^{-4}$ mole to about $2.39 \times 10^2$ moles of pMB, or from about $3.77 \times 10^{-4}$ mole to about $1.39 \times 10^3$ moles of dCP, respectively, in the chromogen solution for each mole of hydrogen peroxide in the substrate solution. Preferably, the equivalent ratio for each of these reactants is from about $7.5 \times 10^{-3}$ to about $1.9 \times 10^{-2}$ mole of 4-aminoantipyrine and from about 0.15 mole to about 0.36 mole of HDCBS, or from about 0.88 mole to about 2.14 moles of pHBA, or from about 0.019 mole to about 0.045 mole of pMB, or from about 0.038 mole to about 0.091 mole of dCP, respectively, in the chromogen solution for each mole of hydrogen peroxide in the substrate solution.

Preferably, the chromogen solution is lyophilized to a dry powdery form which provides easier storage, increased shelf life and handling convenience than does the solution itself. The lyophilization step is performed in accordance with well-known and conventional methodologies.

The method of the invention may be employed in the form of a convenient testing kit providing a stoppered test tube for the chromogen, either in solution or in lyophilized form, an air-tight container such as a pliable sealed vial or another stopper test tube for the peroxide solution and a calibrated dropper for the saliva sample. The testing kit may contain, for example, a sufficient amount of the buffered chromogen and the peroxide solution for one or more tests. In conducting the test, the peroxide solution is simply added to the chromogen in the test tube followed by a measured sample of saliva, that is, an amount sufficient for the test, previously collected in the dropper to the calibrated measurement. The test tube may then be re-stoppered, shaken and then allowed to stand for the indicated time interval. Means can be provided in the kit for positioning the test tube such that, after the color reaction is completed, the resultant color formation may be immediately compared to a color indicator chart also provided with the kit and from which the absence or presence and the severity of the inflammatory periodontal disease can be determined.

The method of the invention may be employed in the form of a kit for detecting the presence of inflammatory periodontal disease comprising the following separately contained components:

(a) a hydrogen peroxide solution; and

(b) a lyophilized reagent consisting of 4-amino-antipyrine; a phenolic component selected from the group consisting of sodium 2-hydroxy-3,5-dichlorobenzene sulfonate, p-hydroxybenzoic acid, p-methoxyphenol and 2,6-dichlorophenol; and a buffer to provide a pH of about 4—9 upon admixture of said lyophilized reagent with said hydrogen peroxide solution. In place of said lyophilized reagent, the kit may contain the heretofore described aqueous buffered chromogen solution.

Alternate methods include the use of porcelain wells which provide excellent receptacles for performing the assay with small volumes of reactants and a white background for observing the assay color result; as does the use of a suitable absorbent material, for example, commercially available filter support pads cut into convenient shapes for unit testing, e.g. discs, which maintain all the reactants thereon without leakage and from which the assay color result is readily discernible; and the use of conventional spectrophotometric instruments to automatically "color read" the test assay solutions.

The following example is given for the purpose of illustrating the invention.

## Example 1

An independent clinical trial was conducted by a research dentist in order to compare and correlate the clinical results thus obtained with the in-vitro results obtained from the subject assay. Each patient was clinically examined to determine the absence or presence of inflammatory periodontal disease and the severity thereof. The result of each clinical diagnosis was scored from 1 (normal) to 4 (severe). A total of 22 male and female subjects participated in the trial. Each participant was asked to refrain from any oral hygiene or food intake before collecting approximately 5 mls of saliva after awakening in the morning. Each patient was asked to spit once into a spital collecting tube. Each of the saliva samples were analyzed to

4

detect the presence of the peroxidase enzyme according to the following test tube method.

*Test Tube Method:* The test reaction was carried out at room temperature in the following manner:

a. 50 microliters of the Preferred Chromogen Solution was lyophilized in a test tube;

b. 110 microliters of the Preferred Substrate Solution was added to the lyophilized chromogen;

c. 30 microliters of the saliva test sample was added to initiate the reaction;

d. 30 minutes after the saliva addition, the presence or absence and intensity of the developed color due to the presence of peroxidase enzyme in the saliva was scored on the aforementioned scale of 1 to 4.

The results of the clinical diagnosis in the clinical trial and the results of the in-vitro saliva assay for each of the 22 subjects are shown in Table I.

## TABLE I

| Patient Number | In Vitro Score | | | | In-Vivo Clinical Score |
|---|---|---|---|---|---|
| | HDCBS | pHBA | pMP | dCP | |
| 1 | 2 | 2 | 2 | 2 | 1 |
| 2 | 1 | 1 | 1 | 1 | 2 |
| 3 | 2 | 2 | 1 | 2 | 1-2 |
| 4 | 1 | 1 | 1 | 1 | 1-2 |
| 5 | 3 | 3 | 2 | 2 | 1 |
| 6 | 1 | 1 | 1 | 1 | 2 |
| 7 | 3 | 3 | 3 | 2 | 1 |
| 8 | 3 | 3 | 3 | 2 | 1-2 |
| 9 | 4 | 4 | 4 | 4 | 4 |
| 10 | 1 | 1 | 1 | 1 | 2 |
| 11 | 3 | 3 | 2 | 3 | 1 |
| 12 | 1 | 1 | 1 | 1 | 2 |
| 13 | 2 | 3 | 3 | 2 | 1 |
| 14 | 3 | 3 | 3 | 3 | 1 |
| 15 | 1 | 1 | 1 | 1 | 1 |
| 16 | 1 | 1 | 1 | 1 | 2-3 |
| 17 | 1 | 1 | 1 | 1 | 1 |
| 18 | 1 | 1 | 2 | 1 | 2-3 |
| 19 | 3 | 3 | 2 | 3 | 1 |
| 20 | 1 | 1 | 1 | 1 | 1 |
| 21 | 1 | 1 | 1 | 1 | 1 |
| 22 | 2 | 2 | 2 | 2 | 2-3 |

## Claims

1. A method for detecting the presence of inflammatory periodontal disease in a human suspected of being afflicted with same which comprises admixing a saliva sample from such human with an aqueous solution of 4-aminoantipyrine and a phenolic component selected from the group consisting of sodium 2-hydroxy-3,5-dichlorobenzene sulfonate (HDCBS), p-hydroxybenzoic acid (pHBA), p-methoxyphenol (pMP), and 2,6-dichlorophenol (dCP) buffered to a pH about 4—9 and with hydrogen peroxide solution and observing the color produced as an indicator of such presence.

2. The method of Claim 1 wherein said phenolic component is 2-hydroxy-3,5-dichlorobenzene sulfonate (HDCBS).

3. The method of Claim 1 wherein said phenolic component is p-hydroxybenzoic acid (pHBA).

4. The method of Claim 1 wherein said phenolic component is p-methoxyphenol (pMP).

5. The method of Claim 1 wherein said phenolic component is 2,6-dichlorophenol (dCP).

6. A method for detecting the presence of inflammatory periodontal disease in a human suspected of being afflicted with same which comprises admixing a unit volume of saliva sample from such human with about 2—15 unit volumes of a chromogen solution consisting essentially of:

|  | % w/v |
|---|---|
| 4-aminoantipyrine | 0.001 — 0.15 g |
| phenolic component: | |
| if HDCBS | 0.07 — 1.0 g |
| if pHBA | 0.10 — 15.0 g |
| if pMP | 4.1 — 130.0 mg |
| if dCP | 6.5 — 1000.0 mg |
| KH$_2$PO$_4$ | 0.15 — 12.0 g |
| K$_2$HPO$_4$ | 0.15 — 13.0 g |
| Water | 100 ml |

and with about 0.1—2 unit volumes of a substrate solution consisting essentially of:

|  | % v/v |
|---|---|
| hydrogen peroxide (30%) | 0.001 — 10 ml |
| water, q.s. | 100 ml |

and observing the color produced as an indicator of such presence.

7. The method of Claim 6 wherein said chromogen solution consists essentially of:

|  | % w/v |
|---|---|
| 4-aminoantipyrine | 0.017 g |
| sodium 2-hydroxy-3,5-dichloro-benzene sulfonate (HDCBS) | 0.42 g |
| KH$_2$PO$_4$ (monobasic) | 5.98 g |
| K$_2$HPO$_4$ (dibasic) | 6.27 g |
| water, q.s. | 100 ml |

and said substrate solution consists essentially of:

|  | % v/v |
|---|---|
| hydrogen peroxide (30%) | 0.55 ml |
| water, q.s. | 100 ml |

8. The method of Claim 6 wherein said chromogen solution consists essentially of:

|  | % w/v |
|---|---|
| 4-aminoantipyrine | 0.017 g |
| p-hydroxybenzoic acid (pHBA) | 1.5 mg |
| KH$_2$PO$_4$ (monobasic) | 5.98 g |
| K$_2$HPO$_4$ (dibasic) | 6.27 g |
| water, q.s. | 100 ml |

6

and said substrate solution consists essentially of:

|  | % v/v |
| --- | --- |
| hydrogen peroxide (30%) | 0.55 ml |
| water, q.s. | 100 ml |

9. The method of Claim 6 wherein said chromogen solution consists essentially of:

|  | % w/v |
| --- | --- |
| 4-aminoantipyrine | 0.017 g |
| p-methoxyphenol (pMP) | 25.0 g |
| $KH_2PO_4$ (monobasic) | 5.98 g |
| $K_2HPO_4$ (dibasic) | 6.27 g |
| water, q.s. | 100 ml |

and said substrate solution consists essentially of:

|  | % v/v |
| --- | --- |
| hydrogen peroxide (30%) | 0.55 ml |
| water, q.s. | 100 ml |

10. The method of Claim 6 wherein said chromogen solution consists essentially of:

|  | % w/v |
| --- | --- |
| 4-aminoantipyrine | 0.017 g |
| 2,6-dichlorophenol (dCP) | 25.0 mg |
| $KH_2PO_4$ (monobasic) | 5.98 g |
| $K_2HPO_4$ (dibasic) | 6.27 g |
| water, q.s. | 100 ml |

and said substrate solution consists essentially of:

|  | % v/v |
| --- | --- |
| hydrogen peroxide (30%) | 0.55 ml |
| water, q.s. | 100 ml |

**Patentansprüche**

1. Verfahren zur Feststellung der Anwesenheit von entzündlicher Parodontal-Erkrankung bei einem Menschen, bei dem Verdacht besteht, daß diese bei ihm vorliegt, umfassend das Vermischen einer Speichelprobe dieses Menschen mit einer wäßrigen Lösung von 4-Aminoantipyrin und einer phenolischen Komponente, die ausgewählt ist aus Natrium-2-hydroxy-3,5-dichlorbenzolsulfonat (HDCBS), p-Hydroxybenzoesäure (p-HBA), p-Methoxyphenol (pMP) und 2,6-Dichlorphenol (dCP), gepuffert auf einen pH-Wert von etwa 4 bis 9, und mit einer Wasserstoffperoxidlösung, und Feststellung der erzeugten Färbung als Indikator einer solchen Gegenwart.

2. Verfahren nach Anspruch 1, wobei die phenolische Komponente 2-Hydroxy-3,5-dichlorbenzol-sulfonat (HDCBS) ist.

3. Verfahren nach Anspruch 1, wobei die phenolische Komponente p-Hydroxybenzoesäure (pHBA) ist.

4. Verfahren nach Anspruch 1, wobei die phenolische Komponente p-Methoxyphenol (pMP) ist.

7

5. Verfahren nach Anspruch 1, wobei die phenolische Komponente 2,6-Dichlorphenol (dCP) ist.

6. Verfahren zur Feststellung der Anwesenheit von entzündlicher Parodontal-Erkrankung bei einem Menschen, bei dem Verdacht besteht, daß diese bei ihm vorliegt, umfassend das Vermischen einer Volumeneinheit einer Speichelprobe dieses Menschen mit etwa 2 bis 15 Volumeneinheit einer chromogenen Lösung, die im wesentlichen besteht aus

| | % Gew./Vol. |
|---|---|
| 4-Aminoantipyrin | 0,001 - 0,15 g |
| phenolische Komponente: | |
| falls HDCBS | 0,007 - 1,0 g |
| falls pHBA | 0,10 - 15,0 g |
| falls pMP | 4,1 - 130,0 mg |
| falls dCP | 6,5 - 1000,0 mg |
| $KH_2PO_4$ | 0,15 - 12,0 g |
| $K_2HPO_4$ | 0,15 - 13,0 g |
| Wasser | 100 ml |

und mit etwa 0,1 bis 2 Volumeneinheiten einer Substratlösung, die im wesentlichen besteht aus

| | % Vol./Vol. |
|---|---|
| Wasserstoffperoxid (30%) | 0,001 - 10 ml |
| Wasser auf | 100 ml |

und Feststellung der erzeugten Färbung als Indikator für eine solche Gegenwart.

7. Verfahren nach Anspruch 6, wobei die chromogene Lösung im wesentlichen besteht aus:

| | % Gew./Vol. |
|---|---|
| 4-Aminoantipyrin | 0,017 g |
| Natrium-2-hydroxy-3,5-dichlor-benzolsulfonat (HDCBS) | 0,42 g |
| $KH_2PO_4$ (monobasisch) | 5,98 g |
| $K_2HPO_4$ (dibasisch) | 6,27 g |
| Wasser auf | 100 ml |

und die Substratlösung im wesentlichen besteht aus:

| | % Vol./Vol. |
|---|---|
| Wasserstoffperoxid (30 %) | 0,55 ml |
| Wasser auf | 100 ml. |

EP 0 205 844 B1

8. Verfahren nach Anspruch 6, wobei die chromogene Lösung im wesentlichen besteht aus:

|  | % Gew./Vol. |
|---|---|
| 4-Aminoantipyrin | 0,017 g |
| p-Hydroxybenzoesäure (pHBA) | 1,5 mg |
| $KH_2PO_4$ (monobasisch) | 5,98 g |
| $K_2HPO_4$ (dibasisch) | 6,27 g |
| Wasser auf | 100 ml |

und die Substratlösung im wesentlichen besteht aus

|  | % Vol./Vol. |
|---|---|
| Wasserstoffperoxid (30 %) | 0,55 ml |
| Wasser auf | 100 ml |

9. Verfahren nach Anspruch 6, wobei die chromogene Lösung im wesentlichen besteht aus:

|  | % Gew./Vol. |
|---|---|
| 4-Aminoantipyrin | 0,017 g |
| p-Methoxyphenol (pMP) | 25,0 mg |
| $KH_2PO_4$ (monobasisch) | 5,98 g |
| $K_2HPO_4$ (dibasisch) | 6,27 g |
| Wasser auf | 100 ml |

und die Substratlösung im wesentlichen besteht aus

|  | % Vol./Vol. |
|---|---|
| Wasserstoffperoxid (30 %) | 0,55 ml |
| Wasser auf | 100 ml |

10. Verfahren nach Anspruch 6, wobei die chromogene Lösung im wesentlichen besteht aus:

|  | % Gew./Vol. |
|---|---|
| 4-Aminoantipyrin | 0,017 g |
| 2,6-Dichlorphenol (dCP) | 25,0 mg |
| $KH_2PO_4$ (monobasisch) | 5,98 g |
| $K_2HPO_4$ (dibasisch) | 6,27 g |
| Wasser auf | 100 ml |

und die Substratlösung im wesentlichen besteht aus

|  | % Vol./Vol. |
|---|---|
| Wasserstoffperoxid (30 %) | 0,55 ml |
| Wasser auf | 100 ml |

**Revendications**

1. Procédé de détection de la présence d'une maladie périodontale inflammatoire chez un être humain suspecté d'en être affligé, comprenant un mélange d'un échantillon de salive de cet être humain à une solution aqueuse de 4-aminoantipyrine et d'un composant phénolique choisi parmi le groupe constitué de sulfonate de 2-hydroxy-3,5-dichlorobenzène sodique (HDCBS), d'acide p-hydroxybenzoïque (pHBA), de p-méthoxyphénol (pMP) et de 2,6-dichlorophénol (dCP), tamponnée à un pH d'environ 4—9 et avec une

9

solution de peroxyde d'hydrogène, et une observation de la couleur produite, comme indicateur de cette présence.

2. Procédé suivant la revendication 1, caractérisé en ce que le composant phénolique est du sulfonate de 2-hydroxy-3,5-dichlorobenzène (HDCBS).

3. Procédé suivant la revendication 1, caractérisé en ce que le composant phénolique est de l'acide p-hydroxybenzoïque (pHBA).

4. Procédé suivant la revendication 1, caractérisé en ce que le composant phénolique est du p-méthoxyphénol (pMP).

5. Procédé suivant la revendication 1, caractérisé en ce que le composé phénolique est du 2,6-dichlorophénol (dCP).

6. Procédé de détection de la présence d'une maladie périodontale inflammatoire chez un être humain soupçonné d'en être affligé, comprenant un mélange d'un volume unitaire d'échantillon de salive de cet être humain à environ 2 à 15 volumes unitaires d'une solution chromogène constituée essentiellement de:

| | % poids/volume |
|---|---|
| 4-aminoantipyrine | 0,001 - 0,15 g |
| composant phénolique : | |
| si HDCBS | 0,07 - 1,0 g |
| si pHBA | 0,10 - 15,0 g |
| si pMP | 4,1 - 130,0 mg |
| si dCP | 6,5 - 1000,0 mg |
| $KH_2PO_4$ | 0,15 - 12,0 g |
| $K_2HPO_4$ | 0,15 - 13,0 g |
| Eau | 100 ml |

et à environ 0,1—2 volumes unitaires d'une solution de substrat constituée essentiellement de:

| | % vol./vol. |
|---|---|
| peroxyde d'hydrogène (30 %) | 0,001 - 10 ml |
| eau, q.s. | 100 ml |

et une observation de la couleur produite, comme indicateur de cette présence.

7. Procédé suivant la revendication 6, caractérisé en ce que la solution chromogène est constituée essentiellement de:

| | % poids/vol. |
|---|---|
| 4-aminoantipyrine | 0,017 g |
| Sulfonate de 2-hydroxy-3,5-dichlorobenzène sodique (HDCBS) | 0,42 g |
| $KH_2PO_4$ (monobasique) | 5,98 g |
| $K_2HPO_4$ (dibasique) | 6,27 g |
| eau, q.s. | 100 ml |

et en ce que la solution de substrat est essentiellement constituée de:

| | % vol./vol. |
|---|---|
| peroxyde d'hydrogène (30 %) | 0,55 ml |
| eau, q.s. | 100 ml |

10

8. Procédé suivant la revendication 6, caractérisé en ce que la solution chromogène est essentiellement constituée de:

| | % poids/vol. |
|---|---|
| 4-aminoantipyrine | 0,017 g |
| acide p-hydroxybenzoïque (pHBA) | 1,5 mg |
| $KH_2PO_4$ (monobasique) | 5,98 g |
| $K_2HPO_4$ (dibasique) | 6,27 g |
| eau, q.s. | 100 ml |

et en ce que la solution de substrat est essentiellement constituée de:

| | % vol./vol. |
|---|---|
| peroxyde d'hydrogène (30 %) | 0,55 ml |
| eau, q.s. | 100 ml |

9. Procédé suivant la revendication 6, caractérisé en ce que la solution chromogène est essentiellement constituée de:

| | % poids/vol. |
|---|---|
| 4-aminoantipyrine | 0,017 g |
| p-méthoxyphénol (pMP) | 25,0 g |
| $KH_2PO_4$ (monobasique) | 5,98 g |
| $K_2HPO_4$ (dibasique) | 6,27 g |
| eau, q.s. | 100 ml |

et en ce que la solution de substrat est essentiellement constituée de:

| | % vol./vol. |
|---|---|
| peroxyde d'hydrogène (30 %) | 0,55 ml |
| eau, q.s. | 100 ml |

10. Procédé suivant la revendication 6, caractérisé en ce que la solution chromogène est essentiellement constituée de:

| | % poids/vol. |
|---|---|
| 4-aminoantipyrine | 0,017 g |
| 2,6-dichlorophénol (dCP) | 15,0 mg |
| $KH_2PO_4$ (monobasique) | 5,98 g |
| $K_2HPO_4$ (dibasique) | 6,27 g |
| eau, q.s. | 100 ml |

et en ce que la solution de substrat est essentiellement constituée de:

| | % vol./vol. |
|---|---|
| peroxyde d'hydrogène (30 %) | 0,55 ml |
| eau, q.s. | 100 ml |

11